# FASCICULE DE BREVET EUROPEEN

(11) **EP 2 689 754 B1**
(45) Date de publication et mention de la délivrance du brevet: **03.02.2016**
(21) Numéro de dépôt: 13177724.5
(22) Date de dépôt: 24.07.2013
(51) Int. Cl.: A61F 5/01

(54) **Dispositif de protection et de correction de l'Hallux Valgus**
Schutz- und Korrekturvorrichtung von Hallux Valgus
Device for protecting and correcting hallux valgus

(30) Priorité: 26.07.2012 FR 1257244
(43) Date de publication de la demande: 29.01.2014
(73) Titulaire: Vitry Freres, 75116 Paris (FR)
(72) Inventeur: De Grandcourt, Frédéric, 44650 Touvois (FR)
(74) Mandataire: Regimbeau

(56) Documents cités:
- BE-A3- 1 018 634
- DE-C- 602 101
- FR-A1- 2 883 458
- GB-A- 887 330
- GB-A- 2 477 281
- JP-U- H0 548 920
- US-A1- 2011 046 531

## Description

La présente invention concerne le traitement d'une malformation du gros orteil dénommée Hallux Valgus.

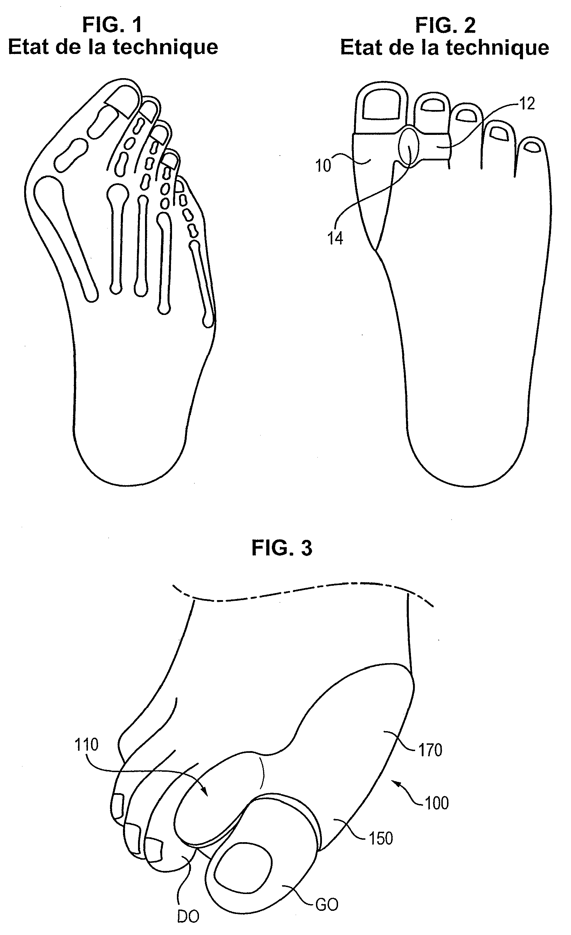

L'Hallux Valgus, du latin hallus qui signifie « gros orteil » et valgus qui signifie « tourné en dehors », qualifie une déformation du gros orteil vers l'extérieur tel que schématisé sur la figure 1 annexée.

Cette malformation ou déformation s'accompagne souvent d'un oignon ou callosité de la peau en regard de la déformation, sur le côté intérieur du pied.

Les effets premiers principaux générés sont des douleurs sur le bord interne du pied en regard de la tête du métatarsien, de possibles inflammations et parfois ulcérations au niveau des zones de la peau entrant en conflit avec la chaussure et la gêne au chaussage du fait que la bosse induite oblige à porter des chaussures larges et souvent sans talon.

L'Hallux Valgus peut également conduire à des effets secondaires conséquents parmi lesquels on ne citera ici que le déplacement associé des tendons extenseurs et fléchisseurs du gros orteil et de ce fait une force axiale réduite sur l'articulation, une déformation de l'ongle du gros orteil et une déformation en griffe résultante du deuxième orteil.

De très nombreux moyens ont déjà été proposés pour tenter de traiter l'Hallux Valgus.

A titre d'exemples non limitatifs, on citera :
- des moyens le plus souvent d'ancrage, nécessitant des interventions chirurgicales, tels que proposés dans les documents US2012/0071935, WO2011/160107, US2012/0016428, US2011/077656, WO2011/153417, WO2011/091166,
- des systèmes de type semelles orthopédiques, chaussures de rétention ou modules de protection ou soulagement, tels que proposés dans les documents WO2012/64127, KR2012/0032093, WO2011/092645, WO2012/28921, FR 2883458,
- des équipements de type manchons ou bandes installés à la demande sur le pied à traiter, tels que décrits dans les documents DE2008-004213, FR2793406, DE9206317, JP2011-130819, JP2007-313045, US5497789, DE4315085, CN201879878, JP3-188849, JP2964405, US5453083, WO2011/11019, ou encore

- des dispositifs de correction tels que décrits par exemple dans les documents CN2008-178436, CN2018/22942, CN2149879, WO2011/086716, JP 2002-520034, JP2012-000168, JP2007-244786, JP2011-245007.

Les systèmes semble-t-il les plus usités aujourd'hui, sont formés de manchons de protection du genre décrit dans le document FR2793406.

Ces systèmes ne donnent cependant pas totalement satisfaction.

Les systèmes du type précité sont en effet peu confortables, complexes à installer car d'appareillage difficile et difficile à chausser, et d'efficacité réduite car leurs effets dépendent en grande partie de la tension donnée par l'utilisateur à des sangles de maintien.

Un autre système est connu du document JPH0548920U. Ce système comprend un insert destiné à être placé entre le gros orteil et le deuxième orteil et un fourreau venu de matière avec ledit insert et adapté pour recevoir le gros orteil. Le dispositif est réalisé d'une pièce en élastomère thermoplastique. L'insert s'étend essentiellement en avant dudit fourreau, et le fourreau est prolongé par une coupelle sur l'arrière à l'opposé de l'insert. L'insert comprend une partie centrale de section droite en forme de goutte d'eau.

La présente invention a pour objectif de proposer de nouveaux moyens de traitement de l'Hallux Valgus qui permettent d'éliminer les inconvénients de la technique antérieure.

Ce but est atteint dans le cadre de la présente invention grâce à un dispositif de protection et de correction de l'Hallux Valgus tel que défini dans la revendication 1 annexée.

Le dispositif de protection et de correction conforme à la présente invention se distingue en particulier d'un dispositif décrit dans le document JP2011-130819 schématisé sur la figure 2 annexée. Le dispositif décrit dans le document JP2011-130819 comprend deux anneaux 10, 12, reliés par un élément de liaison intermédiaire 14. Un tel dispositif n'a pas connu le développement escompté dans la mesure où en pratique sa pose s'avère trop difficile pour la plupart des patients.

D'autres caractéristiques, buts et avantages de la présente invention apparaîtront à la lecture de la description détaillée qui va suivre et en regard des dessins annexés, donnés à titre d'exemple non limitatif et sur lesquels :
- la figure 1 précédemment décrite représente schématiquement l'anatomie d'un pied révélant un Hallux Valgus,
- la figure 2 précédemment décrite représente un dispositif conforme au document antérieur JP2011-130819,
- la figure 3 représente schématiquement un dispositif de protection et de correction de l'Hallux Valgus, conforme à la présente invention, installé sur le pied d'un patient,

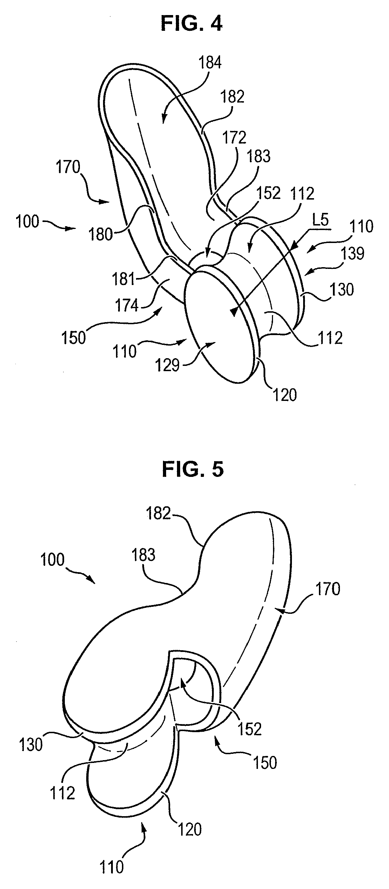

- les figures 4 et 5 représentent deux vues en perspective du dispositif de protection et de correction de l'Hallux Valgus conforme à la présente invention,
- la figure 6 représente une vue interne en plan d'un dispositif de protection et de correction conforme à la présente invention,
- la figure 7 représente une vue schématique en coupe transversale du même dispositif de protection et de correction conforme à la présente invention selon le plan de coupe schématisé VII-VII sur la figure 8,
- la figure 8 représente une vue latérale extérieure du dispositif de protection et de correction conforme à la présente invention, et
- la figure 9 représente une vue en coupe longitudinale du dispositif de protection et de correction de l'Hallux Valgus conforme à la présente invention selon le plan de coupe référencé IX-IX sur la figure 6.

Le dispositif 100 de protection et de correction de l'Hallux Valgus conforme à la présente invention est formé d'une pièce comprenant, venus de matière, un insert 110 destiné à être placé entre le gros orteil GO et le deuxième orteil DO comme on le voit sur la figure 3 et un fourreau 150 adapté pour recevoir le gros orteil GO, ledit fourreau 150 étant prolongé vers l'arrière, soit à l'opposé de l'insert 110, par une coupelle 170 incurvée concave vers l'intérieur et destinée à recouvrir l'oignon formé au niveau de la bosse résultant de la déformation du gros orteil, comme on le voit également sur la figure 3.

Comme indiqué précédemment, le dispositif 100 conforme à la présente invention est formé d'une pièce unique, sans ajout de matière, notamment sans ajout de surépaisseur par exemple sans revêtement de tissu additionnel.

Le dispositif 100 conforme à la présente invention est réalisé en élastomère thermoplastique, tel qu'un polyuréthane souple.

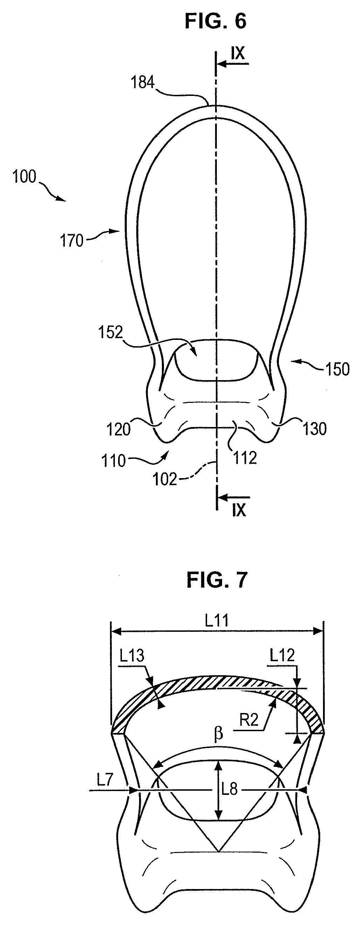

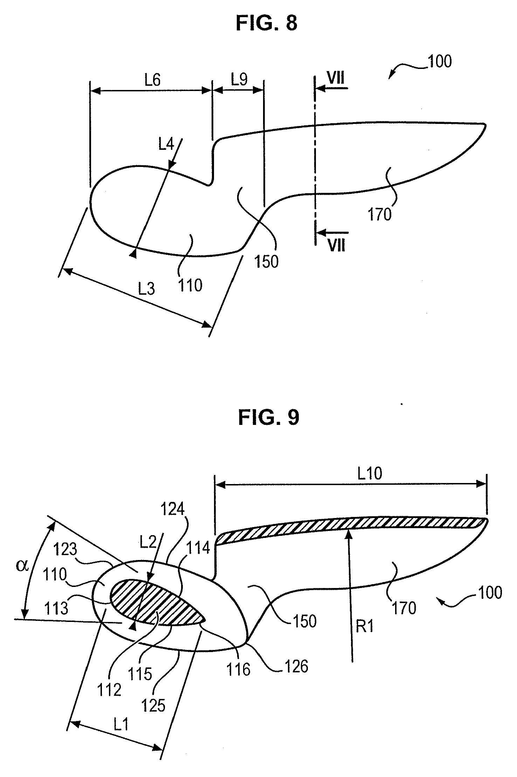

Le dispositif 100 présente de préférence une symétrie par rapport à un plan longitudinal 102 référencé sur la figure 6 et qui coïncide avec le plan de coupe de la figure 9. Ainsi, le dispositif 100 conforme à la présente invention est ambidextre, c'est-à-dire qu'il peut être placé indifféremment sur un pied gauche ou un pied droit. Cependant, en variante, l'on peut prévoir une légère dissymétrie du dispositif afin de permettre une parfaite adaptation à certaines anatomies spécifiques.

L'insert 110 a la forme générale d'un osselet ou astragale. Il est constitué d'un volume plein. Ainsi l'insert tout en présentant une certaine souplesse, présente une forte résistance à l'écrasement.

Plus précisément, l'insert 110 comprend une partie centrale 112 qui s'évase progressivement de part et d'autre du plan de symétrie 102 sous forme de deux flasques latéraux 120, 130.

La partie centrale 112 présente dans le plan de symétrie 102 une section droite en forme de goutte d'eau, comme on le voit sur la figure 9.

Dans le plan de symétrie 102, la section de la partie centrale 112 est ainsi délimitée à l'avant par un tronçon formé d'un secteur de cylindre 113, prolongé vers l'arrière par deux facettes sensiblement planes 114, 115, qui convergent en direction de l'arrière du dispositif pour se rejoindre au niveau d'une pointe arrière globalement arrondie 116.

Le rayon de courbure du tronçon circulaire 113 est de préférence compris entre 5 et 9mm, avantageusement de l'ordre de 7mm.

La plus grande longueur L1 de la section droite de la partie centrale 112, dans le plan de symétrie 102, est de préférence comprise entre 15 et 35mm et de préférence de l'ordre de 25mm.

La hauteur L2 de la section de la partie centrale 112, dans le plan de symétrie 102est de préférence comprise entre 5 et 15mm et de préférence de l'ordre de 10mm.

L'angle α de convergence des deux facettes 114, 115 est de préférence de l'ordre de 30°.

Comme indiqué précédemment, la partie centrale 112 de l'insert 110 s'évase progressivement de part et d'autre du plan de symétrie 102 en éloignement de celui-ci pour aboutir sur les flasques 120, 130. La section droite des flasques 120, 130, considérée parallèlement au plan de symétrie 102 est globalement homothétique de la section droite dans le plan de symétrie 102. Le contour des flasques 120, 130 est ainsi également délimité par une calotte cylindrique 123 sur son extrémité avant, laquelle calotte cylindrique 123 est prolongée vers l'arrière par deux facettes 124, 125, globalement rectilignes qui convergent vers l'arrière pour se rejoindre au niveau d'une pointe globalement arrondie 126.

Le rayon de courbure de la calotte cylindrique avant 123 est de préférence compris entre 10 et 25mm, avantageusement de l'ordre de 17mm. La plus grande longueur L3 des flasques 120, 130, considérée parallèlement au plan de symétrie 102, est de préférence comprise entre 30 et 50mm, avantageusement de l'ordre de 35mm, tandis que la hauteur L4 des flasques 120, 130, considérée également parallèlement au plan de symétrie 102, est de préférence comprise entre 15 et 25mm, avantageusement de l'ordre de 19mm.

L'épaisseur L5 de l'insert 110 considéré perpendiculairement au plan de symétrie 102 est de préférence comprise entre 20 et 35mm, avantageusement de l'ordre de 25mm.

Les faces extérieures 129, 139 des flasques 120, 130 sont de préférence globalement planes et parallèles au plan de symétrie 102.

L'angle moyen de convergence des facettes 124, 125 des flasques 120, 130 est globalement identique à l'angle de convergence α précité des facettes 114, 115 de la partie centrale 110.

Lorsque l'on observe l'insert 110 selon une vue axiale longitudinale, comme illustré sur les figures 6 et 7, la transition entre la section dans le plan de symétrie 102 visible sur la figure 9 et le plus grand encombrement des flasques latéraux 120, 130 est une évolution de courbure continue, sans discontinuité, en forme générale de U.

Le fourreau ou anneau 150 est solidaire de l'extrémité arrière de l'insert 110. Une partie de sa périphérie est délimitée par la courbure en U précitée de l'insert 110 formée par l'évolution entre la section dans le plan de symétrie 102 de plus faible contour et les flasques 120, 130 de plus grande dimension.

De ce fait, comme indiqué précédemment, et ceci constitue une caractéristique essentielle vis-à-vis de l'enseignement du document JP2011-130819, selon la présente invention l'insert 110 s'étend essentiellement en avant du fourreau 150.

Plus précisément, selon l'invention, l'insert 110 dépasse en avant du fourreau 150 d'une longueur L6 au moins égale à 10mm, de préférence au moins égale à 15mm et très avantageusement au moins de l'ordre de 20mm.

Le fourreau 150 peut posséder au repos une ouverture centrale 152 (destinée à recevoir le gros orteil GO) ayant une section droite globalement circulaire de révolution. Cependant, selon l'invention, le fourreau ou anneau 150 présente au repos une ouverture centrale 152 de section droite ovoïde ayant son grand axe transversal au plan de symétrie 102.

Au repos, le fourreau 150 peut posséder un grand axe L7 compris entre 15 et 25mm, de préférence de l'ordre de 20mm, tandis que le petit axe L8 du fourreau est compris entre 7 et 20mm, de préférence de l'ordre de 12mm.

Le fourreau 150 possède de préférence une largeur L9 considérée parallèlement au plan de symétrie 102 , comprise entre 6 et 20mm, de préférence de l'ordre de 12mm.

Le fourreau 150 est prolongé vers l'arrière par une coupelle 170 en forme générale de cuiller concave. La coupelle 170 prolonge l'enveloppe du fourreau 150 de sorte que la coupelle 170 peut être considérée comme raccordée à l'insert 110 par deux bras 172, 174 raccordés latéralement sur la partie arrière des deux flasques 120, 130 sur la tranche de ceux-ci.

Dans le plan de symétrie 102, la coupelle 170 est définie par une génératrice présentant un rayon de courbure R1 de l'ordre de 50cm.

La longueur L10, dans le plan de symétrie 102, du cumul du fourreau 150 et de la coupelle 170 est avantageusement comprise entre 40 et 80mm, préférentiellement de l'ordre de 60mm. Ainsi dans le plan de symétrie, la flèche de la génératrice délimitant la coupelle 170 est faible, typiquement comprise entre 1 et 6mm, avantageusement de l'ordre de 2 à 3mm.

Comme on le voit sur la figure 7, dans un plan perpendiculaire au plan de symétrie 102, la génératrice délimitant la coupelle 170 possède un rayon de courbure R2 typiquement compris entre 15 et 40mm, avantageusement de l'ordre de 33mm.

Dans le plan de coupe de la figure 7, l'angle d'ouverture β de la coupelle 170 est de l'ordre de 80°.

Comme on le voit sur les figures, cette ouverture angulaire de la coupelle 170 se réduit progressivement en direction de son extrémité arrière. Les bords latéraux 180, 182 de la coupelle convergent progressivement, sans discontinuité, vers l'arrière pour se rejoindre sous forme d'un contour arrondi au niveau de la pointe la plus arrière 184 de la coupelle.

La largeur L11 de la coupelle 170, considérée perpendiculairement au plan de symétrie 102 sensiblement à mi longueur de la coupelle 170, étant de préférence comprise entre 20 et 55mm, avantageusement de l'ordre de 35mm, la flèche L12 à mi-longueur de la coupelle 170 est avantageusement comprise entre 4 et 15mm, de préférence de l'ordre de 7mm.

L'épaisseur L13 du dispositif conforme à l'invention est sensiblement constante au niveau du fourreau 150 et de la coupelle 170. Plus précisément, au niveau du fourreau 150 et de la coupelle 170 cette épaisseur est de préférence comprise entre 1 et 5mm, avantageusement de l'ordre de 3mm.

L'ensemble des bords de la coupelle sont de préférence globalement chanfreinés et arrondis.

De même, la transition entre les bords latéraux 180, 182 de la coupelle et le fourreau 150 est de préférence une transition concave continue référencée 181, 183.

Outre que l'insert 110 s'étend essentiellement sur l'avant du fourreau 150, le dispositif de protection et de correction de l'Hallux Valgus conforme à la présente invention se distingue de l'objet divulgué dans le document JP2011-130819 par le fait qu'il ne comporte qu'un seul fourreau et non pas deux anneaux destinés à être placés sur le gros orteil et le deuxième orteil.

Cette différence est essentielle car elle facilite l'engagement sur le pied de tout sujet, tout particulièrement de personnes âgées.

Le dispositif conforme à la présente invention offre ainsi l'énorme avantage d'une conception qui permet un usage alliant simplicité d'application et confort dû à la matière qui permet d'épouser parfaitement la surface de contact de la zone de pied concernée, sans créer la moindre surépaisseur.

Le dispositif conforme à la présente invention offre l'avantage sous forme d'une pièce unique, du double effet à la fois de la protection de l'Hallux Valgus et du redressement du gros orteil grâce à l'insert 110.

La protection de l'oignon est définie par l'anneau 150 et la coupelle 170, celle-ci étant parfaitement maintenue en position par l'anneau 150 qui s'engage sur le gros orteil et l'insert 110 qui sert de stabilisateur et de positionnement. L'élément intermédiaire formé par l'insert 110 qui se projette en avant de l'anneau 150 contribue par son positionnement au redressement du gros orteil.

Plus précisément, l'insert 110 destiné à être placé entre le gros orteil et le deuxième orteil, est selon l'invention, sans lien avec le troisième orteil. Ainsi le gros orteil n'est pas aligné avec les autres orteils, mais complètement repoussé vers l'extérieur de façon autonome. L'insert 110 formant redresseur aide ainsi à réaligner le gros orteil et à le soulager en le mettant au repos. L'insert soulage ainsi la tension créée par l'Hallux Valgus, en alignant de façon précise le gros orteil, en repoussant la tension vers l'extérieur du pied et non pas vers l'intérieur, sans être poussé vers les autres orteils du pied.

Dans le cadre de l'invention, l'insert 110 dépasse à l'avant du fourreau 150 d'une longueur supérieure à la largeur du fourreau 150, et de préférence d'une longueur au moins de l'ordre de grandeur de deux fois la largeur du fourreau 150. Par ailleurs cette longueur de dépassement de l'insert 110 sur l'avant du fourreau 150 est de préférence proche de la moitié de la longueur de la coupelle 170.

Le dispositif 100 réalisé de préférence en élastomère thermoplastique, tel qu'un polyuréthane souple, peut être moulé d'une pièce en un matériau globalement transparent ou être teinté dans la masse, par exemple pour avoir une couleur chair.

Les valeurs dimensionnelles mentionnées précédemment sont données à titre d'exemple et ne peuvent être considérées comme limitatives.

Des pièces de différentes tailles conformes à la présente invention peuvent être réalisées afin de permettre une bonne adaptation à la morphologie de chaque individu.

Dans un contexte simple mais non limitatif on peut ainsi concevoir un jeu de trois dispositifs respectivement de taille petite, moyenne et grande. Un nombre supérieur de tailles peut être réalisé si l'on souhaite améliorer l'adaptation à chaque patient.

## Revendications

1. Dispositif de protection et de correction de l'Hallux Valgus comprenant un insert (110) destiné à être placé entre le gros orteil et le deuxième orteil et un fourreau (150) venu de matière avec ledit insert (110) et adapté pour recevoir le gros orteil, le dispositif étant réalisé d'une pièce en élastomère thermoplastique, l'insert (100) s'étendant essentiellement en avant dudit fourreau (150), le fourreau (150) étant prolongé par une coupelle (170) sur l'arrière à l'opposé de l'insert (110), dans lequel l'insert (110) a la forme d'un osselet ou astragale comprenant une partie centrale de section droite en forme de goutte d'eau s'évasant progressivement sous forme de deux flasques (120, 130) de plus grand encombrement.

2. Dispositif selon la revendication 1, réalisé en polyuréthane.

3. Dispositif selon l'une des revendications 1 ou 2, dans lequel la longueur de la coupelle (170) est comprise entre 40 et 80mm, avantageusement de l'ordre de 60mm.

4. Dispositif selon l'une des revendications 1 à 3, dans lequel l'insert (110) a dans son plan médian une longueur comprise entre 15 et 35mm, avantageusement de l'ordre de 25mm et une hauteur comprise entre 5 et 15mm, avantageusement de l'ordre de 10mm.

5. Dispositif selon l'une des revendications 1 à 4, dans lequel le fourreau (150) possède une section droite ovoïde au repos.

6. Dispositif selon l'une des revendications 1 à 5, dans lequel l'insert (110) dépasse du fourreau (150) d'une longueur au moins égale à 10mm, avantageusement supérieure à 15mm et très préférentiellement au moins de l'ordre de 20mm.

7. Dispositif selon l'une des revendications 1 à 6, présentant un plan de symétrie longitudinal.

8. Dispositif selon l'une des revendications 1 à 7, dans lequel l'insert (110) dépasse à l'avant du fourreau (150) d'une longueur supérieure à la largeur du fourreau (150), et de préférence d'une longueur au moins de l'ordre de grandeur de deux fois la largeur du fourreau (150) et cette longueur de dépassement de l'insert (110) sur l'avant du fourreau (150) est de préférence proche de la moitié de la longueur de la coupelle (170).

9. Dispositif selon l'une des revendications 1 à 8, dans lequel l'insert (110) a la forme générale d'un osselet ou astragale constitué d'un volume plein.

10. Dispositif selon l'une des revendications 1 à 9, ne comportant qu'un seul fourreau (150) adapté pour recevoir le gros orteil et non pas deux anneaux destinés à être placés respectivement sur le gros orteil et le deuxième orteil.

## Patentansprüche

1. Schutz- und Korrekturvorrichtung des Hallux Valgus, umfassend einen Einsatz (110), der dazu bestimmt ist, zwischen dem großen Zeh und dem zweiten Zeh platziert zu werden, und eine Hülse (150), die aus demselben Material wie der genannte Einsatz (110) gebildet ist und geeignet ist, um den großen Zeh aufzunehmen, wobei die Vorrichtung aus einem Stück aus thermoplastischem Elastomer realisiert ist, wobei sich der Einsatz (100) im Wesentlichen an der Vorderseite der genannten Hülse (150) erstreckt, wobei die Hülse durch eine Kupelle (170) an der Rückseite gegenüber dem Einsatz (110) verlängert ist, in dem der Einsatz (110) die Form eines Knöchelchens oder Rundstabes hat, umfassend einen zentralen Teil mit geradem Querschnitt in Tropfenform, die sich progressiv in Form von zwei Flanschen (120, 130) mit größerem Platzverbrauch aufweitet.

2. Vorrichtung gemäß Anspruch 1, die in Polyurethan realisiert ist.

3. Vorrichtung gemäß Anspruch 1 oder 2, bei der die Länge der Kupelle (170) zwischen 40 und 80 mm, vorteilhaft in der Größenordnung von 60 mm inbegriffen ist.

4. Vorrichtung gemäß Anspruch 1 bis 3, bei der der Einsatz (110) in seiner medianen Ebene eine zwischen 15 und 35 mm inbegriffene, vorteilhaft in der Größe von 25 mm hat und eine zwischen 5 und 15 mm inbegriffene Höhe, vorteilhaft in der Größenordnung von 10 mm hat.

5. Vorrichtung gemäß Anspruch 1 bis 4, bei der die Hülse (150) einen geraden, eiförmigen Querschnitt in Ruhestellung hat.

6. Vorrichtung gemäß Anspruch 1 bis 5, bei der der Einsatz (110) aus der Hülse (150) um einen Länge von wenigstens gleich 10 mm, vorteilhaft mehr als 15 mm und sehr vorteilhaft wenigstens in der Größenordnung von 20 mm hervorsteht.

7. Vorrichtung gemäß Anspruch 1 bis 6, die eine symmetrische Längsebene aufweist.

8. Vorrichtung gemäß Anspruch 1 bis 7, bei der der Einsatz (110) aus der Vorderseite der Hülse (150) um eine größere Länge als die Breite der Hülse (150) und bevorzugt um eine Länge von wenigstens in der Größenordnung von zwei Mal der Breite der Hülse (150) hervorsteht und diese Hervorstehlänge des Einsatzes (110) auf der Vorderseite der Hülse (150) bevorzugt nahe der Hälfte der Länge der Kupelle (170) ist.

9. Vorrichtung gemäß Anspruch 1 bis 8, bei der der Einsatz (110) die allgemeine Form eines Knöchelchens oder Rundstabs hat, der aus einem massiven Volumen gebildet ist.

10. Vorrichtung gemäß Anspruch 1 bis 9, umfassend nur eine einzige Hülse (150), die geeignet ist, um den großen Zeh aufzunehmen, und nicht zwei Ringe, die dazu bestimmt sind, jeweils auf dem großen Zehn und dem zweiten Zeh platziert zu werden.

## Claims

1. A device for protecting and correcting Hallux Valgus comprising an insert (110) intended to be placed between the big toe and the second toe and a casing (150) provided in a single piece with said insert (110) and adapted to accommodate the big toe, the device being integrally made of a thermoplastic elastomer, the insert (100) substantially extending at the front of said casing (150), the casing (150) being continued by a cup (170) at the back opposite the insert (110), wherein the insert (110) is in the shape of an ossicle or talus comprising a water drop-shaped centre part of cross-section gradually flaring as two flanges (120, 130) of larger overall dimensions.

2. The device according to claim 1, made of polyurethane.

3. The device according to one of claims 1 or 2, wherein the length of the cup (170) is between 40 and 80mm, advantageously in the order of 60mm.

4. The device according to one of claims 1 to 3, wherein the insert (110) in its mid-plane has a length between 15 and 35mm, advantageously in the order of 25mm and a height between 5 and 15mm, advantageously in the order of 10mm.

5. The device according to one of claims 1 to 4, wherein the casing (150) has an ovoid cross-section at rest.

6. The device according to one of claims 1 to 5, wherein the insert (110) protrudes from the casing (150) by a length at least equal to 10mm, advantageously greater than 15mm and most preferably at least in the order of 20mm.

7. The device according to one of claims 1 to 6, exhibiting a longitudinal plane of symmetry.

8. The device according to one of claims 1 to 7, wherein the insert (110) protrudes at the front of the casing (150) by a greater length than the width of the casing (150), and preferably by a length at least in the order of magnitude of twice the width of the casing (150) and this protruding length of the insert (110) at the front of the casing is preferably close to half the length of the cup (170).

9. The device according to one of claims 1 to 8, wherein the insert (110) is in the general shape of an ossicle or talus made up of a solid volume.

10. The device according to one of claims 1 to 9, only including one casing (150) adapted to accommodate the big toe and not two rings intended to be placed on the big toe and the second toe, respectively.
